(19)

**Europäisches Patentamt**
**European Patent Office**
**Office européen des brevets**

(11) **EP 1 458 748 B1**

(12) **EUROPÄISCHE PATENTSCHRIFT**

(45) Veröffentlichungstag und Bekanntmachung des Hinweises auf die Patenterteilung:
**20.07.2011 Patentblatt 2011/29**

(51) Int Cl.:
***C07K 7/06*** *(2006.01)*

(21) Anmeldenummer: **02805332.0**

(22) Anmeldetag: **18.12.2002**

(86) Internationale Anmeldenummer:
**PCT/EP2002/014439**

(87) Internationale Veröffentlichungsnummer:
**WO 2003/054009 (03.07.2003 Gazette 2003/27)**

(54) **APOPTOTISCH WIRKSAME PEPTIDE**

APOPTOTICALLY ACTIVE PEPTIDES

PEPTIDES AYANT UN EFFET SUR L'APOPTOSE

(84) Benannte Vertragsstaaten:
**AT BE BG CH CY CZ DE DK EE ES FI FR GB GR IE IT LI LU MC NL PT SE SI SK TR**

(30) Priorität: **20.12.2001 DE 10163130**

(43) Veröffentlichungstag der Anmeldung:
**22.09.2004 Patentblatt 2004/39**

(60) Teilanmeldung:
**10010931.3 / 2 332 964**

(73) Patentinhaber: **CytoTools AG**
**64285 Darmstadt (DE)**

(72) Erfinder:
- **FREYBERG, Mark, Andre**
  **64285 Darmstadt (DE)**
- **FRIEDL, Peter**
  **64287 Darmstadt (DE)**
- **KAISER, Dirk**
  **64287 Darmstadt (DE)**

(74) Vertreter: **Schultheiss, Jürgen**
**Patentanwalt**
**Postfach 24 01 30**
**55045 Mainz (DE)**

(56) Entgegenhaltungen:
**WO-A-00/45856     WO-A-00/47102**
**WO-A-96/11942     WO-A-96/17059**
**US-A- 5 399 667**

- **DATABASE GENBANK [Online] 29. Mai 1998 (1998-05-29) FLEISCHMANN R. D. ET AL.: "Haemophilus influenzae Rd section 148 of 163 of the complete genome" Database accession no. U32833 XP002248148**
- **DATABASE GENBANK [Online] 19. September 1995 (1995-09-19) FRIEDBERG D. ET AL.: "Salmonella typhimurium leucine-responsive regulatory protein (Lrp) gene, complete cds" Database accession no. U02273 XP002248149**
- **DATABASE GENBANK [Online] 1. Juni 2000 (2000-06-01) HARTMANN E. & GOERLICH D.: "Drosophila melanogaster Ran binding protein 11 (Ranbp11) mRNA, complete cds" Database accession no. AF245515 XP002248150**
- **DATABASE GENBANK [Online] 17. Dezember 2001 (2001-12-17) STAPLETON M. ET AL.: "Drosophila melanogaster LD41918 full length cDNA" Database accession no. AY069650 XP002248151**
- **DATABASE GENBANK [Online] READ T. ET AL.: "Bacillus anthracis str. Ames section 1 of 18 of the complete genome" Database accession no. ae017024 XP002248152**
- **DATABASE GENBANK [Online] 25. Mai 2000 (2000-05-25) TETTELIN H. ET AL.: "Neisseria meningitidis serogroup B strain MC58 section 157 of 206 of the complete genome" Database accession no. AE002515 XP002248153**
- **DATABASE GENBANK [Online] 11. März 1998 (1998-03-11) DUBOIS E. ET AL.: "S. cerevisiae chomosome II reading frame ORF YBR222c" Database accession no. Z36091 XP002248154**

**(Forts. nächste Seite)**

- DATABASE GENBANK [Online] 17. März 1994 (1994-03-17) LEE Y. A. ET AL.: "Xanthomonas campestris juglandis copper-resistance genes, complete cds" Database accession no. L19222 XP002248155
- DATABASE GENBANK [Online] 3. Juni 2000 (2000-06-03) NAKAJIMA M. ET AL.: "Pseudomonas syringae plasmid pPaCu1 ORFA, ORFB, ORFC, ORFD genes, complete cds" Database accession no. AB033419 XP002248156
- DATABASE GENBANK [Online] 25. Mai 2000 (2000-05-25) TETTELIN H. ET AL.: "Neisseria meningitidis serogroup B strain MC58 section 36 to 206 of the complete genome" Database accession no. AE002394 XP002248157
- DATABASE GENBANK [Online] 3. November 2001 (2001-11-03) LEE L. F. ET AL.: "Streptomyces lividans ebrC repressor (ebrS) and multidrug resistance efflux protein (ebrC) genes, complete cds" Database accession no. AY043331 XP002248158
- DATABASE EMBL [Online] 1. November 2001 (2001-11-01) SKEIKY Y. A. W. ET AL.: "Propionibacterium acnes immunogenic protein #19049" Database accession no. AAU58153 XP002248159
- CARRON J A ET AL: "A CD36-binding peptide from thrombospondin-1 can stimulate resorption by osteoclasts in vitro." BIOCHEMICAL AND BIOPHYSICAL RESEARCH COMMUNICATIONS, Bd. 270, Nr. 3, 21. April 2000 (2000-04-21), Seiten 1124-1127, XP002248144 ISSN: 0006-291X
- GAO AI-GUO ET AL: "Integrin-associated protein is a receptor for the C-terminal domain of thrombospondin." JOURNAL OF BIOLOGICAL CHEMISTRY, Bd. 271, Nr. 1, 1996, Seiten 21-24, XP002248145 ISSN: 0021-9258
- GAO A-G ET AL: "THROMBOSPONDIN MODULATES ALPHAVBETA3 FUNCTION THROUGH INTEGRIN-ASSOCIATED PROTEIN" THE JOURNAL OF CELL BIOLOGY, ROCKEFELLER UNIVERSITY PRESS, US, Bd. 135, Nr. 2, Oktober 1996 (1996-10), Seiten 533-544, XP000990435 ISSN: 0021-9525
- KOSFELD MINH D ET AL: "Identification of a new cell adhesion motif in two homologous peptides from the COOH-terminal cell binding domain of human thrombospondin." JOURNAL OF BIOLOGICAL CHEMISTRY, Bd. 268, Nr. 12, 1993, Seiten 8808-8814, XP002248146 ISSN: 0021-9258
- JIMENEZ BENILDE ET AL: "Signals leading to apoptosis-dependent inhibition of neovascularization by thrombospondin-1." NATURE MEDICINE., Bd. 6, Nr. 1, Januar 2000 (2000-01), Seiten 41-48, XP002248147 ISSN: 1078-8956

SEQUENZPROTOKOLL

<110> CytoTools GmbH

<120> Apoptotisch wirksame Peptide

<130> C 688wo

<160> 19

<170> PatentIn version 3.1

<210> 1
<211> 6
<212> PRT
<213> Künstliche Sequenz

<220>
<223> Synthetisches Peptid

<400> 1

```
                          Arg Ala Tyr Val Val Met
                          1               5
```

<210> 2
<211> 6
<212> PRT
<213> Künstliche Sequenz

<220>
<223> Synthetisches Peptid

<400> 2

```
                          Arg Trp Tyr Val Val Met
                          1               5
```

<210> 3
<211> 6
<212> PRT

<213> Künstliche Sequenz

<220>
<223> Synthetisches Peptid

<400> 3

```
                              Arg Tyr Tyr Val Val Met
                              1               5
```

<210> 4
<211> 6
<212> PRT
<213> Künstliche Sequenz

<220>
<223> Synthetisches Peptid

<400> 4

```
                              Arg Glu Tyr Val Val Met
                              1               5
```

<210> 5
<211> 10
<212> PRT
<213> Künstliche Sequenz

<220>
<223> Synthetisches Peptid

<400> 5

```
                    Lys Arg Ala Tyr Val Val Met Trp Lys Lys
                    1               5                   10
```

<210> 6
<211> 10
<212> PRT
<213> Künstliche Sequenz

<220>
<223> Synthetisches Peptid

<400> 6

```
                    Lys Arg Glu Tyr Val Val Met Trp Lys Lys
                    1               5                   10
```

<210> 7
<211> 6
<212> PRT
<213> Künstliche Sequenz

<220>
<223> Synthetisches Peptid

<400> 7

```
Arg Gly Tyr Val Val Met
1               5
```

<210> 8
<211> 6
<212> PRT
<213> Künstliche Sequenz

<220>
<223> Synthetisches Peptid

<400> 8

```
Arg Met Tyr Val Val Met
1               5
```

<210> 9
<211> 6
<212> PRT
<213> Künstliche Sequenz

<220>
<223> Synthetisches Peptid

<400> 9

```
Arg Thr Tyr Val Val Met
1               5
```

<210> 10
<211> 6
<212> PRT
<213> Künstliche Sequenz

<220>
<223> Synthetisches Peptid

<400> 10

```
                              Arg Asn Tyr Val Val Met
                              1               5
```

<210> 11
<211> 6
<212> PRT
<213> Künstliche Sequenz

<220>
<223> Synthetisches Peptid

<400> 11

```
                              Arg Asp Tyr Val Val Met
                              1               5
```

<210> 12
<211> 6
<212> PRT
<213> Künstliche Sequenz

<220>
<223> Synthetisches Peptid

<400> 12

```
                              Met Val Val Tyr Phe Arg
                              1               5
```

<210> 13
<211> 6
<212> PRT
<213> Künstliche Sequenz

<220>
<223> Synthetisches Peptid

<400> 13

```
                              Arg Ala Tyr Val Val Ala
                              1               5
```

<210> 14
<211> 6
<212> PRT
<213> Künstliche Sequenz

<220>
<223> Synthetisches Peptid

<400> 14

```
                              Arg Leu Tyr Val Val Met
                              1               5
```

<210> 15
<211> 6
<212> PRT
<213> Künstliche Sequenz

<220>
<223> Synthetisches Peptid

<400> 15

```
                         Arg Pro Tyr Val Val Met
                         1               5
```

<210> 16
<211> 6
<212> PRT
<213> Künstliche Sequenz

<220>
<223> Synthetisches Peptid

<400> 16

```
                              Arg Ser Tyr Val Val Met
                              1               5
```

<210> 17
<211> 6
<212> PRT
<213> Künstliche Sequenz

<220>
<223> Synthetisches Peptid

<400> 17

```
                              Arg Cys Tyr Val Val Met
                              1               5
```

<210> 18
<211> 6
<212> PRT
<213> Künstliche Sequenz aus D-Aminosäuren

<220>
<223> Synthetisches Peptid

<400> 18

```
met val val tyr phe arg
1                   5
```

<210> 19
<211> 6
<212> PRT
<213> Künstliche Sequenz aus D-Aminosäuren

<220>
<223> Synthetisches Peptid

<400> 19

```
met val val tyr ala arg
1                   5
```

**Beschreibung**

Die vorliegende Erfindung betrifft apoptotisch wirksame Peptide sowie deren Verwendung zur Herstellung von Medikamenten.

Apoptose ist ein genetisch kodiertes Selbstmordprogramm, welches in eukaryontischen Zellen unter bestimmten physiologischen oder pathologischen Bedingungen induziert wird.

Es werden pathologische Zustände diskutiert, bei denen die Apoptose negative Auswirkungen hat, und zu deren Behandlung die Apoptose inhibiert werden muss.

Ein Beispiel für eine solche Krankheit ist die Ateriosklerose. Insbesondere konnte von den Erfindern schon früher gezeigt werden, dass gerade im Bereich der ateriosklerotischen Plaques apoptotische Zellen (besonders: Endothelzellen, glatte Muskelzellen) auftreten, und dass dieses Auftreten durch gestörte Strömungsverhältnisse verstärkt wird, also strömungsabhängig ist (Freyberg et al.,BBRC, 286, 141-149, 2001). Weiterhin konnten die Erfinder zeigen, dass Substanzen, die die Bindung von TSP-1 an IAP und/oder $\alpha_v\beta_3$ inhibieren, auch die strömungsabhängige Apoptose weitgehend inhibieren können.

Es besteht daher ein hoher Bedarf an Substanzen, die die Apoptose negativ beeinflussen. Insbesondere wäre es günstig, die strömungsabhängige und mit der Ateriosklerose ursächlich verbundene Apoptose inhibieren zu können. Weiterhin besteht hoher Bedarf an pharmazeutischen Formulierungen, die solche Substanzen enthalten, und die zur Behandlung von Zuständen eingesetzt werden können, bei denen die Inhibition der Apoptose angezeigt ist, insbesondere zur Behandlung der Arteriosklerose,. Diese Substanzen sollten dabei möglichst von geringem Molekulargewicht und/ oder kleine Peptide sein, um eine gute Bioverfügbarkeit zu gewährleisten.

Aufgabe der vorliegenden Erfindung war es daher, apoptotisch wirksame Substanzen, bevorzugt Peptide, zur Verfügung zu stellen. Insbesondere war es eine weitere Aufgabe der vorliegenden Erfindung, Substanzen, bevorzugt Peptide, zur Verfügung zu stellen, die eine durch TSP-1 induzierte strömungsabhängige Apoptose der Endothelzellen inhibieren können. Eine weitere Aufgabe der vorliegenden Erfindung war es, pharmazeutische Zubereitungen zur Verfügung zu stellen, mit denen Krankheiten behandelt werden können, bei denen die Inhibition von Apoptose angezeigt ist.

Diese und weitere, nicht explizit genannte Aufgaben, die sich aber aus der vorangegangenen Würdigung des Stands der Technik ohne weiteres ergeben, werden durch die in den Ansprüchen definierten Ausführungsbeispiele der vorliegenden Erfindung gelöst.

Insbesondere wird diese Aufgabe durch zur Verfügungstellen von Medikamenten zur Behandlung von Arteriosklerose gemäß Anspruch 1, die Peptide mit einer der in den SEQ ID NOs 1 bis 11 dargestellten Aminosäuresequenzen umfassen.

Unter einem bevorzugten Ausführungsbeispiel der vorliegenden Erfindung handelt es sich dabei um Apoptoseinhibierende Substanzen, die Aminosäuresequenzen der allgemeinen Formel (1):

$$R\text{-}A_1\text{-}Y\text{-}V\text{-}V\text{-}M \text{ umfassen,}$$

wobei $A_1$ für A, D, E, G, M, N, T, W oder Y steht, oder pharmazeutisch annehmbare Salze dieser Substanzen.

Für die Zwecke der vorliegenden Erfindung wird der international übliche Einbuchstabencode für Aminosäuren verwendet, A steht also für Alanin (Ala), C für Cystein (Cys), D für Asparaginsäure (Asp), E für Glutaminsäure (Glu), F für Phenylalanin (Phe), G für Glycin (Gly), L für Leucin (Leu), M für Methionin (Met), N für Asparagin (Asn), P für Prolin (Pro), R für Arginin (Arg), S für Serin (Ser), T für Threonin (Thr), V für Valin (Val), W für Tryptophan (Trp) und Y Für Tyrosin (Tyr). L-Aminosäuren werden hierbei durch Großbuchstaben und D-Aminosäuren durch die Verwendung von Kleinbuchstaben symbolisiert.

Unter einem besonders bevorzugten Gesichtspunkt betrifft die vorliegende Erfindung daher Apoptose inhibierende Substanzen, bevorzugt Peptide, die eine der unter SEQ ID NO 1 bis SEQ ID NO 11 gezeigten Peptidsequenzen umfassen, oder deren entsprechende pharmazeutisch annehmbare Salze.

Weiterhin betrifft die vorliegende Erfindung die Verwendung der erfindungsgemäßen Substanzen, bevorzugt Proteine oder Peptide umfassend mindestens eine der in SEQ ID NO 1 bis SEQ ID NO 11 dargestellten Aminosäuresequenz zur Herstellung von Medikamenten zur Behandlung von Arteriosklerose,.

Überraschenderweise wurde von den Erfindern gezeigt, dass Peptide die eine durch Formel (1) dargestellte Aminosäuresequenz umfassen, in überaus starkem Maße Apoptose inhibieren. Insbesondere sind diese Peptide hervorragend dazu geeignet, die strömungsabhängige, TSP-1 induzierte Apoptose von Endothelzellen zu inhibieren. Es wird jedoch vermutet, dass diese Peptide auch dazu in der Lage sind, bei anderen Zellen die Apoptose zu inhibieren.

Als ganz besonders bevorzugte Ausführungsbeispiele der vorliegenden Erfindung werden folgende Peptide/ Peptidsequenzen zur Verfügung gestellt:

    1. R-A-Y-V-V-M (SEQ ID NO 1)
    2. R-W-Y-V-V-M (SEQ ID NO 2)

3. R-Y-Y-V-V-M (SEQ ID NO 3)
4. R-E-Y-V-V-M (SEQ ID NO 4)
5. K-R-A-Y-V-V-M-W-K-K (SEQ ID NO 5)
6. K-R-E-Y-V-V-M-W-K-K (SEQ ID NO 6)
7. R-G-Y-V-V-M (SEQ ID NO 7)
8. R-M-Y-V-V-M (SEQ ID NO 8)
9. R-T-Y-V-V-M (SEQ ID NO 9)
10. R-N-Y-V-V-M (SEQ ID NO 10)
11. R-D-Y-V-V-M (SEQ ID NO 11)

Für die Zwecke der vorliegenden Erfindung wird unter dem Begriff "Peptid" eine Substanz verstanden, die aus einer Kette von 2 oder mehr, durch Peptidbindungen verbundenen Aminosäuren, besteht. Insbesondere weisen erfindungsgemäße apoptotisch wirksame Peptide eine Kettenlänge von < 100 Aminosäuren, bevorzugt <75, besonders bevorzugt von <50, ganz besonders bevorzugt von <25 und am meisten bevorzugt von <15 Aminosäuren auf.

Besonders bevorzugt werden daher für die erfindungsgemäßen Zwecke Peptide, die eine der SEQ ID NOs1-11 umfassen und entweder am N-terminalen und/oder am C-terminalen Ende jeweils 1 bis 3 zusätzliche Aminosäuren umfassen.

Für die Zwecke der vorliegenden Erfindung wird unter "apoptotisch wirksam" verstanden, dass der Zusatz der entsprechenden Substanz in das in den Beispielen 5 und 6 gezeigte Testsystem einen positiven oder negativen Inhibitionsindex erzeugt. Insbesondere werden hierfür Endothelzellen, besonders bevorzugt HUVEC, in Medium kultiviert, welches 1$\mu$g TSP-1 pro ml enthält. Die prozentualen Apoptosewerte dieser Positivkontrolle dienen dann wie in den Beispielen gezeigt der Berechnung des Inhibitionsindex für die Substanzen, die als potentielle Inhibitoren zusammen mit dem Induktor TSP-1 in parallelen Untersuchungen eingesetzt werden. Eine Negativkontrolle zeigt das Fehlen sonstiger Induktoren an.

Ein positiver Inhibitionsindex zeigt dabei an, dass die entsprechende Substanz die Apoptose inhibiert. Ein negativer Inhibitionsindex zeigt dabei an, dass die verwendete Substanz die Apoptose induziert, d.h. die TSP-1 induzierte Apoptose verstärkt.

Native Peptide weisen allerdings oft eine geringe metabolische Stabilität gegenüber Peptidasen und eine relativ schlechte Bioverfügbarkeit auf.

Ausgehend von den oben gezeigten Peptiden kann der Fachmann ohne erfinderisches Zutun eine ganze Reihe von abgeleiteten Verbindungen entwickeln, die eine ähnliche oder gleichartige Wirkungsweise besitzen, und die u.a. auch Peptidmimetica genannt werden.

Als Peptidmimetica werden dabei für die Zwecke der vorliegenden Erfindung Verbindungen bezeichnet, welche die Struktur von Peptiden nachahmen und als Liganden in der Lage sind, die biologische Aktivität auf Rezeptor-/Enzymebene zu imitieren (Agonist) oder zu blockieren (Antagonist). Vor allem sollen die Peptidmimetica eine verbesserte Bioverfügbarkeit und eine verbessert metabolische Stabilität aufweisen. Die Art der Mimetisierung kann von der leicht veränderten Ausgangsstruktur bis zum reinen Nichtpeptid reichen. Siehe beispielsweise A. Adang et al., Recl. Trav. Chim. Pays-Bas 113 (1994), 63-78.

Im Prinzip bieten sich folgende Möglichkeiten zur Mimetisierung/Derivatisierung einer Peptidstruktur an:

- Verwendung von D - statt L -Aminosäuren
- Modifizierung der Seitenkette von Aminosäuren
- Veränderung/Verlängerung der Peptidhauptkette
- Cyclisierung zur Konformationsstabilisierung
- Verwendung von Templaten, die eine bestimmte Sekundärstruktur erzwingen
- Verwendung eines nicht peptidischen Rückgrates, dass mit geeigneten Resten/Seitenketten die Struktur des Peptides nachahmt

Während die proteolytische Stabilität eines Peptids durch Austausch von L- gegen D-Aminosäuren gesteigert werden kann, führt die Modifikation der Seitenketten einer der Aminosäuren oft zu einer Verbesserung der Bindungseigenschaften des gesamten Peptids.

Bei der Veränderung des Peptidrückgrates erfolgt in der Regel ein Austausch einer Amidgruppe gegen amidähnliche Gruppierungen (J. Gante, Angew. Chem. 106 (1994), 1780-1802). Durch diese Maßnahmen lassen sich sowohl die Bindungsaffinität als auch die metabolische Stabilität des nativen Peptids beeinflussen.

Durch die Cyclisierung eines linearen Peptids wird dessen Flexibilität und damit dessen globale Konformation festgelegt. Bei Fixierung der biologisch aktiven Konformation wird die Affinität des Peptids zum Rezeptor erhöht, da die Entropieabnahme bei der Bindung kleiner ist als bei der Bindung eines flexiblen, linearen Peptids. Zu diesem Zweck werden Aminosäureseitenketten, die nicht an der Rezeptorerkennung beteiligt sind, miteinander oder mit dem Peptidgerüst verknüpft.

Die Sekundärstruktur des Peptids spielt eine entscheidende Rolle für die molekulare Erkennung des Rezeptors. Neben α-Helix und β-Faltblatt sind sogenannte Turns als Wendepunkte in der Peptidkette wichtige Konformationselemente. Der Ersatz dieser Struktureinheiten durch einen Baustein, welcher nach dem Einfügen in ein Peptid eine definierte Sekundärstruktur stabilisiert, hat zum Konzept des Sekundärstrukturmimeticum geführt.

Auch die Wasserlöslichkeit der Peptide kann beispielsweise durch Einführung von S- und C- Glycopeptid Derivaten erhöht werden. Weitere Maßnahmen können beispielsweise die PEGylierung der Peptide sein.

Auch die Lipophilie der Hexapeptide kann erhöht werden, indem beispielsweise Phenylalanine an die Peptidsequenz angehängt werden.

Die Cyclisierung bzw. die N-terminale Modifikation von Peptiden wird z.B. von Borchard, Journal of controlled Release 62 (1999), 231 -238 und von Blackwell et al., J. Org. Chem. 10 (2001), 5291-302 beschrieben.

Es ist daher klar, dass der Fachmann ausgehend von dem durch die vorliegende Erfindung vermittelte Wissen mit Leichtigkeit zu einer ganzen Reihe von abgeleiteten Peptidmimetika gelangen kann, die jedoch alle von der vorliegenden Erfindung mitumfasst sind.

Unter einem weiteren bevorzugten Gesichtspunkt stellt die vorliegende Erfindung daher auch Peptidmimetica zur Verfügung, die aus den SEQ ID NOs 1-11 abgeleitetet sind, sowie Substanzen, die solche Peptidmimetica umfassen. Insbesondere stellt die vorliegende Erfindung unter einem weiteren bevorzugten Gesichtspunkt die Verwendung von aus den SEQ ID NOs 1 bis 11 abgeleiteten Peptidmimetica und solche Peptidmimetica umfassenden Substanzen zur Herstellung eines Medikamentes zur Behandlung von Ateriosklerose, zur Verfügung.

Erfindungsgemäße Substanzen, die aktive Bestandteile einer pharmazeutischen Zubereitung sind, werden im Allgemeinen in einem pharmazeutisch annehmbaren Träger gelöst. Beispiele für pharmazeutisch annehmbare Träger können Pufferlösungen wie Phosphatpuffer oder Citratpuffer sein. Zur Aufrechterhaltung der Aktivität der Peptide können auch Reagenzien zugesetzt werden, die pharmazeutisch annehmbar sind und beispielsweise ein reduzierendes Milieu in der pharmazeutischen Zubereitung aufrechterhalten.

Die spezifische Dosierung und Posologie ist für jeden Patienten von einer Anzahl von Faktoren abhängig, einschließlich der Aktivität der verwendeten spezifischen Verbindungen, dem Alter des Patienten, dem Körpergewicht, dem allgemeinen Gesundheitszustand, dem Geschlecht, der Ernährung, der Zeit der Verabreichung, dem Weg der Verabreichung, der Exkretionsrate, der Verbindung mit anderen Arzneimitteln und der Schwere der einzelnen Erkrankung, für die die Therapie angewandt wird. Sie wird von einem Arzt in Abhängigkeit von diesen Faktoren ermittelt.

Peptidmedikamente werden üblicherweise parenteral verabreicht, z.B. durch ein Inhalationsspray, rektal, durch subkutane, intravenöse, intramuskuläre, intraartikuläre und intrathecale Injektions- und Infusionstechniken, oder äußerlich in pharmazeutischen Formulierungen, welche konventionelle, pharmazeutisch annehmbare Träger, Adjuvantien und Vehikel enthalten. Je nach Art der identifizierten Substanz kommen auch andere Verabreichungswege in Betracht, z.B. oral. Bei der Wundheilung werden die erfindungsgemäßen Identifikate bevorzugt in Form von Salben oder Puder verabreicht.

Die Erfindung stellt ebenso pharmazeutische Zusammensetzungen bereit, die eine wirksame Menge einer apoptotisch wirksamen Substanz, bevorzugt eines Peptids, Proteins oder Peptidmimeticums in Kombination mit einem konventionellen pharmazeutischen Träger umfassen. Ein pharmazeutischer Träger ist z.B. ein fester oder flüssiger Füllstoff, ein Verkapselungsmaterial oder ein Lösungsmittel. Beispiele für Materialien, die als pharmazeutische Träger dienen können, sind Zucker wie Lactose, Glucose und Saccharose; Stärke wie Maisstärke und Kartoffelstärke; Cellulose und deren Derivate wie Natriumcarboxymethylcellulose, Ethylcellulose und Celluloseacetat; pulverisierter Tragacanth; Malz; Gelatine; Talg; Arzneimittelträger wie Kakaobutter und Zäpfchenwachse; Öle wie Erdnussöl, Baumwollsamenöl, Färberdistelöl, Sesamöl, Olivenöl, Maisöl und Sojabohnenöl; Polyole wie Propylenglykol, Glycerin, Sorbitol, Mannitol und Polyethylenglykol; Ester wie Ethyloleat und Ethyllaureat; Agar; Puffermittel wie Magnesiumhydroxid und Aluminiumhydroxid; Alginsäure; Pyrogen-freies Wasser; isotonische Salzlösung; Ringers Lösung, Ethylalkohol und Phosphatpufferlösungen wie auch andere nichttoxische kompatible Substanzen, die in pharmazeutischen Formulierungen verwendet werden. Benetzungsmittel, Emulgatoren und Gleitmittel wie Natriumlaurylsulfat und Magnesiumstearat, wie auch Färbemittel, Beschichtungsmittel sowie Parfümierungsmittel und Konservierungsstoffe können ebenso in den Zubereitungen vorhanden sein, entsprechend den Anforderungen des Galenikers. Die Menge des aktiven Wirkstoffes, der mit den Trägermaterialien kombiniert wird, um eine Einzeldosis zu produzieren, wird abhängig von dem behandelten Patienten und der speziellen Methode der Verabreichung variieren.

Pharmazeutisch annehmbare Salze der erfindungsgemäßen Substanzen, bevorzugt Peptide, Proteine oder Peptidmimetica können auf gut bekanntem Weg, beispielsweise durch Lösen der erfindungsgemäßen Verbindungen in der entsprechenden, verdünnten Säure oder Base, z.B. Salzsäure oder Natronlauge, und anschließendem Gefriertrocknen hergestellt werden. Metallsalze können erhalten werden durch Lösen der erfindungsgemäßen Verbindungen in Lösungen, die das entsprechende Ion enthalten, und anschließendem Isolieren der Verbindung über HPLC oder Gelpermeationsverfahren.

Die folgenden Beispiele erläutern die Erfindung näher:

**BEISPIEL 1:** Kultivierung von humanen Endothelzellen aus Nabelschnur-Venen (HUVEC)

Lösungen (steril) :

Kulturmedium: IF-Basalmedium + 15% (v/v) NCS, 5 $\mu$g/ml Transferrin, 5 $\mu$g/ml Heparin, 0,7 $\mu$g/ml FGF, 2 mM L-Glutamin [IF-Basalmedium: 1:1-Mischung aus Iscove's Modifiziertem Dulbecco's Medium (IMDM) und Ham's F12, beide von Life Technologies, Paisley (England)]
NCS: Serum neugeborener Kälber (Sebak, Aidenbach)
FGF: Fibroblastenwachstumsfaktor (eigene Herstellung, gereinigt aus Schweinehirn)

Materialien :

Zellkulturgefäße, gelatiniert

Durchführung:

Die Kultivierung von HUVEC erfolgt in gelatinebeschichteten Kulturgefäßen bei 37°C, 5% $CO_2$ und Wasserdampf-gesättigter Atmosphäre. Das Kulturmedium wird alle 2-3 Tage gewechselt; bei Konfluenz werden die Zellen mit einer Teilungsrate von 1:3 bis 1:5 passagiert. HUVEC wachsen streng kontaktinhibiert und bilden einschichtige Zellrasen mit der typischen Kopfsteinpflastermorphologie. Bei Konfluenz erreichen die Kulturen Zelldichten von 4-9 x $10^4$ Zellen/cm$^2$. Für Apoptoseuntersuchungen werden ausschließlich HUVEC-Kulturen der Passagen 1-4 verwendet.

*Beschichtung von Kulturgefäßen:*

Lösungen (steril) :

Gelatinelösung, 1 % (w/v) in Milli-Q-Wasser
1 g Gelatine (zellkulturgetestet) in 100 ml Milli-Q-Wasser suspendieren, durch Autoklavieren für 20 min bei 121°C und 2 bar lösen und bei Raumtemperatur lagern.
PBS (140 mM NaCl, 3 mM KCl, 8 mM $Na_2HPO_4$, 1,5 mM $KH_2PO_4$)
8 g/l NaCl
0,2 g/l KCl
1,44 g/l $Na_2HPO_4$ x 2 $H_2O$
0,2 g/l $KH_2PO_4$
Die Salze in einem entsprechenden Volumen Milli-Q-Wasser lösen, 20 min bei 121°C und 2 bar autoklavieren und bei Raumtemperatur lagern. Der pH-Wert wird überprüft und liegt zwischen 7,2 und 7,4.

Materialien :

Zellkulturgefäße

Durchführung:

Für die Kultivierung adhärent wachsender Zellen werden Kulturgefäße mit Gelatine beschichtet. Der Boden der Zellkulturgefäße wird mit steriler Gelatinelösung bedeckt, die Zellkulturgefäße werden 15 min bei Raumtemperatur belassen. Die Gelatinelösung wird abgesaugt, die Zellkulturgefäße werden einmal mit PBS gewaschen und können so verwendet werden.

*Subkultivierung adhärenter Zellen*

Lösungen (steril):

PBS
Trypsin/EDTA-Lösung
0,05% (w/v) Trypsin und 0,02% (w/v)EDTA in PBS auflösen und sterilfiltrieren.

Materialien:

Zellkulturgefäße, gelatiniert

Durchführung:

Die Zellen werden mit Trypsin/EDTA-Lösung von der Kulturfläche abgelöst. Das Kulturmedium wird abgesaugt, der Boden des Kulturgefäßes kurz mit PBS gewaschen und mit Trypsin/EDTA-Lösung (~1 ml für eine 25 cm$^2$-Kulturflasche) bedeckt. Die Enzymlösung wird sofort wieder abgesaugt, so dass ein dünner Flüssigkeitsfilm auf den Zellen verbleibt. Die Zellen werden 1-10 min bei Raumtemperatur belassen und das Ablösen der Zellen unter dem Mikroskop verfolgt. Das Ablösen der Zellen kann durch sanftes Aufschlagen des Kulturgefäßes auf der Kante beschleunigt werden. Die Zellen werden in frischem Kulturmedium aufgenommen, eventuell gezählt und in neue Kulturgefäße ausgesät.

**BEISPIEL 2:** Bestimmung der Apoptoserate durch Färbung apoptotischer Zellen mit DAPI

DAPI gehört zur Indolfarbstoffgruppe und ist ein selektiver DNS-Farbstoff. Der Farbstoff wird bei 340-360 nm angeregt, und das Emissionsmaximum liegt bei 480 nm. Er wird für Apoptoseuntersuchungen eingesetzt [vgl. Cohen et al., Immunology Today, 14, No. 3, 126-130 (1993)].

*Morphologische Auswertung:*

Lösungen:

PBS
Formaldehydlösung
4% (v/v) Formaldehyd in PBS
DAPI-Lösung (Molecular Probes, Leiden, Niederlande)
2 $\mu$g/ml DAPI in Methanol

Materialien:

Petrischale (35 mm) mit Zellen in Kultur

Durchführung:

Der Kulturüberstand einer Petrischale wird abgesaugt, der Zellrasen wird 15 Minuten mit 1 ml Formaldehydlösung auf Eis fixiert, zweimal mit 2 ml PBS gewaschen, für 15 Minuten mit 0,5 ml DAPI-Lösung versetzt, mit PBS gewaschen und unter dem Fluoreszenzmikroskop ausgewertet. Es wird mit dem UV-Filtersatz und einem 20 x oder 40 x Objektiv gearbeitet. 500-1000 Zellen werden zufällig ausgewählt und die Zellen mit apoptotischen Kernen gezählt.
Der Apoptose-Index wird nach folgender Formel berechnet:

Apoptose-Index [%] = Anzahl apoptotischer Zellen/Gesamtzellzahl x 100

*Durchfluß cytometrie:*

Lösungen:

PBS

Medium

Ethanol (p.a.) eisgekühlt (-20°C)
DAPI-Puffer
DAPI-Stammlösung
DAPI-Färbelösung

<u>Durchführung:</u>

Der Kulturüberstand wird abgesaugt, und die Zellen werden, ohne sie mit PBS zu waschen, trypsiniert. Die Zellsuspension wird in Medium aufgenommen, gezählt, bei 800 x g für 5 Minuten zentrifugiert, das Sediment in 0,5 ml IF resuspendiert und in 1,5 ml eiskaltes Ethanol getropft. Die Suspension wird über Nacht bei - 20°C gelagert. Nach erneuter Zentrifugation und Resuspendierung des Sediments in 2 ml PBS folgt eine halbstündige Inkubation bei 37°C, eine weitere Zentrifugation, Resuspendierung des Sediments in 5 ml DAPI-Lösung und Auszählung im Durchflußcytometer bei einer Zählrate von 50-300 Ereignissen pro Sekunde. Die erhaltene Auftragung zeigt einen hohen Gipfel an Zellen in der $G_1$-Phase des Zellzyklus, gefolgt von einer Fraktion von Zellen in der S-Phase (mittlere Fluoreszenzintensitäten) und einem letzten Gipfel hoher Fluoreszenzintensitäten, der die Zellen in der $G_2$-Phase repräsentiert. Apoptotische Zellen erscheinen, bedingt durch die abnehmende absolute DNS-Menge pro Zelle, in einem Sub-$G_1$-Gipfel [Darzynklewicz Z. et al., Cytometry, 13, 795-808 (1992); Zamai L. et al., Cytometry, 14, 891-897 (1993)]. Dies zeigt das Auftreten einer Apoptose unter den gewählten Bedingungen.

**BEISPIEL 3:** Induktion der Apoptose und Testsystem für apoptotisch wirksame Peptide bzw. Proteine bei kultivierten Endothelzellen

Die Zellen werden wie in Beispiel 1 beschrieben kultiviert. Nach dem Erreichen der vollständigen Konfluenz werden die Zellen für den Test eingesetzt. Zunächst wird Thrombospondin 1 (1μg/ml) zu frischem Medium gegeben und mit der Wirkung von frischem Medium auf die Apoptoserate von HUVEC verglichen. Tabelle 1 zeigt, dass die Zugabe von Thrombospondin zu einer signifikanten Erhöhung der Apoptoserate führt. Die beobachtete Apoptose bei Zugabe von frischem Medium wird durch das während der Dauer des Experiments sekretierte Thrombospondin induziert..

**Tabelle 1: Induktion der Apoptose bei HUVEC mit TSP-1**

| Kulturmedium | Apoptoserate (%) nach 24 h |
|---|---|
| frisches Medium | $0,9 \pm 0,1$ |
| frisch + TSP-1 (1 μg/ml) | $3,0 \pm 0,4$ |

**BEISPIEL 4:** Peptidsynthese

Die verwendeten Peptide wurden durch das NMI (Naturwissenschaftliches und Medizinisches Institut an der Uni Tübingen, Reutlingen, Deutschland) nach Vorgaben der CytoTools synthetisiert.

**BEISPIEL 5:** Identifikation von apoptotisch wirksamen Hexamer-Peptiden mit Hilfe des erfindungsgemäßen Verfahrens

Die Zellen werden wie in Beispiel 1 beschrieben kultiviert. Die Zellen werden in die entsprechenden Kulturgefäße (z.B. 24-Lochplatte/0,5 ml pro Kavität) ausgesät und nach dem Erreichen der vollständigen Konfluenz für den Test eingesetzt. Die Zellen werden mit neuem Medium versorgt:

(a) frisches Kulturmedium **[Basisrate der Apoptose],**
(b) frisches Medium mit 1 μg/ml TSP-1 **[Apoptose-induzierende Substanz; Kontrolle],**
(c) Medium (b) + Peptid der SEQ ID NO 1, 1mM
(d) Medium (b) + Peptid der SEQ ID NO 2, 1mM
(e) Medium (b) + Peptid der SEQ ID NO 3, 1mM
(f) Medium (b) + Peptid der SEQ ID NO 4, 1mM
(g) Medium (b) + Peptid der SEQ ID NO 7, 1mM
(h) Medium (b) + Peptid der SEQ ID NO 8, 1mM
(i) Medium (b) + Peptid der SEQ ID NO 9, 1mM
(j) Medium (b) + Peptid der SEQ ID NO 10, 1mM
(k) Medium (b) + Peptid der SEQ ID NO 11, 1mM
(l) Medium (b) + Peptid der SEQ ID NO 12, 1mM
(m) Medium (b) + Peptid der SEQ ID NO 13, 1mM
(n) Medium (b) + Peptid der SEQ ID NO 14, 1mM
(o) Medium (b) + Peptid der SEQ ID NO 15, 1mM
(p) Medium (b) + Peptid der SEQ ID NO 16, 1mM
(q) Medium (b) + Peptid der SEQ ID NO 17, 1mM

(r) Medium (b) + Peptid der SEQ ID NO 18, 1mM
(s) Medium (b) + Peptid der SEQ ID NO 19, 1mM

Nach 24 h Inkubation unter Kulturbedingungen (Beispiel 1) werden die Zellen fixiert, mit DAPI gefärbt und morphologisch unter dem Fluoreszenzmikroskop untersucht bzw. durchflußcytometrisch untersucht. Die apoptotischen Zellen und die Gesamtzellzahl werden bestimmt und der Apoptoseindex berechnet (Prozent apoptotischer Zellen). Die Daten von 3 unabhängigen Experimenten mit der Angabe der Mittelwerte und der Standardabweichung sind in Tabelle 2 angegeben. Inhibitorisch wirksame Peptide weisen erfindungsgemäß einen positiven Inhibitionsindex auf, während induktorische Peptide erfindungsgemäß einen negativen Inhibtionsindex aufweisen.

**Tabelle 2:** Es werden folgende Peptide getestet:

| SEQ ID NO | Aminosäure-Sequenz | Apoptose index [%] | Inhibitions-index [%]* |
|---|---|---|---|
| K | Kontrolle | 3,28 ± 0,55 | |
| (1) | R-A-Y-V-V-M | 0,83 ± 0,24 | +75,3 ± 7,5 |
| (2) | R-W-Y-V-V-M | 1,30 ± 0,26 | +61,9 ± 7,7 |
| (3) | R-Y-Y-V-V-M | 0,85 ± 0,17 | +74,7 ± 5,0 |
| (4) | R-E-Y-V-V-M | 1,04 ± 0,30 | +69,0 ± 8,9 |
| (7) | R-G-Y-V-V-M | 1,83 ± 0,37 | +46,4 ± 11,1 |
| (8) | R-M-Y-V-V-M | 1,84 ± 0,39 | +44,2 ± 11.6 |
| (9) | R-T-Y-V-V-M | 1,86±0,36 | +44,5 ± 9,8 |
| (10) | R-N-Y-V-V-M | 2,06±0,53 | +39,5 ± 15,8 |
| (11) | R-D-Y-V-V-M | 2,12±0,47 | +37,3 ± 14,0 |
| (12) | M-V-V-Y-F-R | 4,40±0,53 | -34,1 ± 6,2 |
| (13) | R-A-Y-V-V-A | 4,98±0,3 | -51,8 ± 6,0 |
| (14) | R-L-Y-V-V-M | 6,41±0,63 | -95,4 ± 6,1 |
| (15) | R-P-Y-V-V-M | 5,67 ± 0,54 | -72,8 ± 6,4 |
| (16) | R-S-Y-V-V-M | 6,86±0,37 | -108,4±3,7 |
| (17) | R-C-Y-V-V-M | 13,97±0,35 | -326,5 ± 3,6 |
| (18) | m-v-v-y-f-r | 14,6±0,75 | -345,1±5,1 |
| (19) | m-v-v-y-a-r | 11,4±0,63 | -247,1±5,4 |
| * positiver Inhibitionsindex = Substanz inhibiert; negativer Inhibitionsindex = Substanz induziert; | | | |

Erfindungsgemäß wird der Inhibitionsindex wie folgt errechnet:

$$- \text{Inhibitionsindex } [\%] = (\text{gemessener Apoptoseindex} \cdot 100 / \text{Apoptoseindexkontrolle}) - 100$$

**BEISPIEL 6:** Verbesserung der Inhibitionswirkung durch Verlängerung der Aminosäurekette

Die Zellkultivierung und die Tests wurden wie in **Beispiel 5** beschrieben durchgeführt. Die verwendeten Peptide sind in Tabelle 3 aufgeführt.

**Tabelle 3: Ergebnisse mit verlängerten Peptidsequenzen**

| SEQ ID NO | Aminosäure-Sequenz | Apoptose -index [%] | Inhibitions-index [%]* |
|---|---|---|---|
| | Kontrolle | 8,73 ± 0,43 | |
| (5) | K-R-A-Y-V-V-M-W-K-K | 0.37 ± 0,22 | +95,8 ± 2,6 |
| (6) | K-R-E-Y-V-V-M-W-K-K | 0,48 ± 0,22 | +94,5 ± 2,5 |
| * positiver Inhibitionsindex = Substanz inhibiert; negativer Inhibitionsindex = Substanz induziert; | | | |

**Patentansprüche**

1. Medikament zur Behandlung von Arteriosklerose, umfassend mindestens eine apoptotisch wirksame Substanz oder ein pharmazeutisch annehmbares Salz der apoptotisch wirksamen Substanz und einen pharmazeutisch annehmbaren Träger, **dadurch gekennzeichnet, dass** die apoptotisch wirksame Substanz mindestens einen Peptidanteil mit einer der in den SEQ ID NOs 1 bis 11 dargestellten Aminosäuresequenzen umfasst und anti-apoptotisch wirksam ist und die apoptotisch wirksame Substanz ein Peptid ist, das eine Kettenlänge von < 100 Aminosäuren aufweist.

2. Medikament gemäß Anspruch 1, **dadurch gekennzeichnet, dass** die apoptotisch wirksame Substanz ein Peptid ist, das eine Kettenlänge von < 50 Aminosäuren aufweist.

3. Medikament gemäß Anspruch 2, **dadurch gekennzeichnet, dass** die apoptotisch wirksame Substanz ein Peptid ist, das eine Kettenlänge von < 25 Aminosäuren aufweist.

**Claims**

1. A pharmaceutical for the treatment of arteriosclerosis, comprising at least one apoptotically active substance or a pharmaceutically acceptable salt of the apoptotically active substance and a pharmaceutically acceptable carrier, **characterized in that** the apoptotically active substance comprises at least one peptide part having one of the amino acid sequences represented in SEQ ID NOs 1 to 11, and is anti-apoptotically active, and the apoptotically active substance is a peptide having a chain length of < 100 amino acids.

2. The pharmaceutical according to claim 1, **characterized in that** the apoptotically active substance is a peptide having a chain length of < 50 amino acids.

3. The pharmaceutical according to claim 2, **characterized in that** the apoptotically active substance is a peptide having a chain length of < 25 amino acids.

**Revendications**

1. Médicament pour le traitement de l'artériosclérose, comprenant au moins une substance à effet apoptotique ou un sel, pharmaceutiquement acceptable, de la substance à effet apoptotique et un substrat pharmaceutiquement acceptable, **caractérisé en ce que** la substance à effet apoptotique comprend au moins une part de peptide avec l'une des séquences d'acides aminés représentées dans les SEQ ID N° 1 à 11, tout en ayant un effet anti-apoptotique, et **en ce que** la substance à effet apoptotique est un peptide présentant une longueur de chaîne de <100 acides aminés.

2. Médicament selon la revendication 1, **caractérisé en ce que** la substance à effet apoptotique est un peptide présentant une longueur de chaîne de <50 acides aminés.

3. Médicament selon la revendication 2, **caractérisé en ce que** la substance à effet apoptotique est un peptide présentant une longueur de chaîne de <25 acides aminés.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **Freyberg et al.** *BBRC,* 2001, vol. 286, 141-149
- **A. Adang et al.** *Recl. Trav. Chim. Pays-Bas,* 1994, vol. 113, 63-78
- **J. Gante.** *Angew. Chem.,* 1994, vol. 106, 1780-1802
- **Borchard.** *Journal of controlled Release,* 1999, vol. 62, 231-238
- **Blackwell et al.** *J. Org. Chem.,* 2001, vol. 10, 5291-302
- **Cohen et al.** *Immunology Today,* 1993, vol. 14 (3), 126-130
- **Darzynklewicz Z. et al.** *Cytometry,* 1992, vol. 13, 795-808
- **Zamai L et al.** *Cytometry,* 1993, vol. 14, 891-897